Europäisches Patentamt

European Patent Office

Office européen des brevets

(11) Numéro de publication: **0 250 304 B1**

(12) **FASCICULE DE BREVET EUROPEEN**

(45) Date de publication de fascicule du brevet: **04.03.92**  (51) Int. Cl.⁵: **A61L 9/04**, A61L 9/01

(21) Numéro de dépôt: **87401328.7**

(22) Date de dépôt: **12.06.87**

(54) **Composition pulvérulente à usage de cendrier et procédé pour sa fabrication.**

(30) Priorité: **16.06.86 FR 8608662**

(43) Date de publication de la demande:
**23.12.87 Bulletin 87/52**

(45) Mention de la délivrance du brevet:
**04.03.92 Bulletin 92/10**

(84) Etats contractants désignés:
**BE CH DE ES FR GB IT LI**

(56) Documents cités:
**WO-A-85/00981**
**GB-A- 2 024 014**
**US-A- 3 921 581**
**US-A- 4 146 566**

(73) Titulaire: **Pointier, Alain Jacques**
**82 Rue Diderot**
**F-92500 Rueil Malmaison(FR)**

(72) Inventeur: **Pointier, Alain Jacques**
**82 Rue Diderot**
**F-92500 Rueil Malmaison(FR)**

(74) Mandataire: **Gorree, Jean-Michel et al**
**Cabinet Plasseraud 84, rue d'Amsterdam**
**F-75009 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne une composition pulvérulente à usage de cendrier, comportant une masse pulvérulente constituée de silice, et elle concerne également un procédé pour sa fabrication.

On connaît déjà diverses compositions pulvérulentes destinées à éviter la diffusion d'odeurs indésirables, notamment des odeurs dégagées par les cendres de tabac, mégots de cigarettes ou de cigares, etc. Ces compositions connues ont généralement pour trait commun de comporter une masse granuleuse qui est imprégnée d'un additif parfumé (documents GB-A-2 024 014 et WO-A-85/00981).

L'inconvénient essentiel de ces compositions connues est qu'on laisse à la seule masse granuleuse la fonction d'absorption des odeurs indésirables, alors que cette masse granuleuse a certes de bonnes propriétés extinctives pour étouffer les parties incandescentes, mais n'a qu'une médiocre efficacité pour absorber les odeurs. Ces produits antérieurs ont donc essentiellement pour aptitude de permettre le dégagement odorant de l'additif parfumé qui vient s'ajouter à l'odeur indésirable, pour masquer celle-ci dans toute la mesure du possible. Il en résulte une atmosphère très alourdie et le mélange d'odeurs qui en découle peut s'avérer peu agréable et être mal supporté

En outre, l'additif parfumé qui est ajouté à la masse granuleuse, dans un produit disponible dans le commerce, conserve ses résidus gras qui provoquent une agglomération des grains et diminuent la propriété coulante de la masse granuleuse ; en outre, ce produit graisse les objets avec lesquels il est mis en contact, ce qui en limite considérablement les conditions d'utilisation.

L'invention vise essentiellement à fournir une composition pulvérulente à usage de cendrier qui ne présente pas les inconvénients des produits actuellement connus et qui réponde mieux aux diverses exigences de la pratique.

A cette fin, l'invention propose un procédé de préparation d'une composition pulvérulente à usage de cendrier, comportant une masse pulvérulente constituée de silice, qui se caractérise par les étapes suivantes :
- on chauffe et on brasse la masse pulvérulente,
- puis on pulvérise, sur cette masse pulvérulente chauffée et brassée, une solution liquide contenant au moins un produit absorbeur d'odeurs, notamment du ricinoléate ou grillocin.

De préférence, le chauffage de la masse pulvérulente s'effectue à une température comprise entre environ 180 et 220˚C, de préférence d'environ 200˚C.

Eventuellement, avant d'effectuer la pulvérisation de la solution liquide, la masse pulvérulente est colorée par trempage et brassage dans un bain colorant liquide contenant des hydroplasts pâteux dilués dans un liquide de dispersion, puis par chauffage à une température comprise entre environ 180 et 220˚C pour l'évaporation de la phase liquide, la pulvérisation précitée de la solution liquide étant faite en fin de l'étape de séchage.

Grâce au procédé conforme à l'invention, on obtient une composition pulvérulente parfaitement sèche et qui conserve donc intégralement sa caractéristique de produit coulant. Cette composition possède un excellent pouvoir d'absorbtion des odeurs de cendres de tabac et de mégots, peut dégager un parfum discret, est esthétiquement agréable, et ne tache pas les objets avec lesquels elle est en contact. Son action est permanente, et non pas momentanée comme les divers aérosols ou les bougies actuellement utilisés dans ce même but. En outre, son coût de fabrication reste modeste et elle peut être obtenue en grandes quantités et de façon automatique par des voies de préparation industrielle simples.

D'un emploi simple, cette composition pulvérulente est destinée aux cendriers individuels collectifs ou spéciaux, tels que ceux utilisés dans l'hôtellerie ou en aviation, en procurant une sécurité contre les incendies en raison de son pouvoir extinctif élevé.

Il est souhaitable de choisir une silice de granulométrie assez fine comprise entre 200 et 400 μm, de préférence de l'ordre de 300 μm, c'est-à-dire une silice de qualité verrerie ayant une granulométrie sélectionnée constante et permanente.

Les adjuvents pulvérisés sur la silice sont présents à raison de 5 à 15 % pour le produit absorbeur d'odeurs, 35 à 45 % pour le parfum et environ 50 % pour le solvant (proportions en poids).

La quantité de parfum incorporée peut être de 4 grammes par litre de solution, à 35-40 % d'extrait, avec divers adjuvants tels qu'un fixateur. A titre d'exemple, on peut utiliser les compositions de parfum suivantes :

Exemple 1 (composition colorée en vert) :

Citral 2 %
Libséa Cubéba 5 %
Terpènes Menthe 2 %
Composition citronnée 71 %
Fixateur 20 %

Exemple 2 (composition colorée en jaune) :

Base jasmin 5 %
Base opopanax 5 %

Base épicée 10 %
Base oeillet 2 %
Arora CX 8 %
Composition fleurie 50 %
Fixateur 20 %

Exemple 3 (composition colorée en bleu) :

Lavande 50-52 10 %
Lavande fine 3,5 %
Absolue lavande fine 10 %
Coumarine 1 %
Fixateur lavande 5,7 %
Benzoate de benzyle 25 %
Renforçateur 2,9 %
Musce ambrette 5,7 %
Fixateur 5,7 %
Composition base 30,5 %

**Revendications**

1. Procédé de préparation d'une composition pulvérulente à usage de cendrier comportant une masse pulvérulente constituée de silice, caractérisé en ce qu'il comprend les étapes qui suivent :
   - on chauffe et on brasse la masse pulvérulente,
   - puis on pulvérise, sur cette masse pulvérulente chauffée et brassée, une solution contenant au moins un produit absorbeur d'odeurs, notamment du ricinoléate ou grillocin.

2. Procédé selon la revendication 1, caractérisé en ce que la solution contient également au moins un parfum sous forme d'extrait sec à 35-40%.

3. Procédé selon la revendication 2, caractérisé en ce que le produit absorbeur d'odeurs est présent dans la proportion en poids de 5 à 15 %, le parfum dans la proportion de 35 à 45 % et au moins un solvant dans la proportion d'environ 50 %.

4. Procédé selon l'une quelconque des revendications 1 à 3, caractérisé en ce que le chauffage de la masse pulvérulente s'effectue à une température comprise entre environ 180 et 220°C, de préférence d'environ 200°C.

5. Procédé selon l'une quelconque des revendications 1 à 4, caractérisé en ce qu'avant d'effectuer la pulvérisation de la solution liquide, la masse pulvérulente est colorée par trempage et brassage dans un bain colorant liquide contenant des hydroplasts pâteux dilués dans un liquide de dispersion, puis par chauffage à une température comprise entre environ 180 et 220°C pour l'évaporation de la phase liquide, la pulvérisation précitée de la solution étant faite en fin de l'étape de séchage.

6. Composition pulvérulente à usage de cendrier, caractérisée en ce qu'elle est préparée par mise en oeuvre du procédé selon l'une quelconque des revendications 1 à 5.

**Claims**

1. Method for the preparation of a powder composition for ashtray use comprising a powder mass constituted by silica, characterized by comprising the following steps :
   - heating and stirring the powder mass,
   - then spraying, onto this heated and stirred powder mass, a solution containing at least one odor-absorbing product, particularly ricinoleate or grillocin.

2. Method according to claim 1, characterized in that the solution contains also at least one perfume in the form of a 35-40 % dry extract.

3. Method according to claim 2, characterized in that the odor-absorbing product is present in the proportion by weight of 5 to 15 %, the perfume in the proportion of 35 to 45 % and at least one solvent in the proportion of about 50 %.

4. Method according to anyone of claims 1 to 3, characterized in that the heating of the powder mass is carried out at a temperature comprised between about 180 and 220°C, preferably about 200°C.

5. Method according to anyone of claims 1 to 4, characterized in that, before carrying out the spraying of the liquid solution, the powder mass is colored by soaking and stirring in a liquid dye bath containing pasty hydroplasts diluted in a dispersion liquid, then by heating to a temperature comprised between about 180 and 220°C for the evaporation of the liquid phase, the aforesaid spraying of the solution being done at the end of the drying step.

6. Powder composition for ashtray use, characterized in that it is prepared by carrying out the method according to anyone of claims 1 to 5.

**Patentansprüche**

1. Verfahren zum Herstellen einer pulverigen, eine aus Silizium bestehende pulverige Masse aufweisenden Zusammensetzung für eine Verwendung in Aschenbechern,
**dadurch gekennzeichnet,**
daß es folgende Schritte aufweist :
- Erhitzen und Umrühren der pulverigen Masse,
- Anschließendes Zerstäuben einer wenigstens ein Gerüche absorbierendes Mittel, insbesondere Rizinoleat oder Grillocin, aufweisenden Lösung über der erhitzten und umgerührten pulverigen Masse.

2. Verfahren nach Anspruch 1,
   **dadurch gekennzeichnet,**
   daß die Lösung zu 35 bis 40% ebenfalls wenigstens ein Parfum in Form eines Trockenextrakts enthält.

3. Verfahren nach Anspruch 2,
   **dadurch gekennzeichnet,**
   daß das Gerüche absorbierende Mittel mit einem Gewichtsanteil von 5 bis 15%, das Parfum mit einem Anteil von 35 bis 45% und wenigstens ein Lösungsmittel mit einem Anteil von etwa 50% enthalten sind.

4. Verfahren nach einem der Ansprüche 1 bis 3,
   **dadurch gekennzeichnet,**
   daß das Erhitzen der pulverigen Masse bei einer Temperatur zwischen etwa 180 zu 220° C, vornehmlich bei etwa 200° C, erfolgt.

5. Verfahren nach einem der Ansprüche 1 bis 4,
   **dadurch gekennzeichnet,**
   daß die pulverige Masse vor dem Zerstäuben der Lösung durch Eintauchen in ein flüssiges Farbbad, das in einer Dispersionsflüssigkeit gelöste teigartige Hydroplaste enthält, und Umrühren, anschließend durch Erhitzen auf eine Temperatur zwischen etwa 180 und 220° C zum Verdampfen des flüssigen Anteils gefärbt wird, wobei das vorgenannte Zerstäuben der Lösung am Ende des Trocknungsschritts erfolgt.

6. Pulverige Zusammensetzung zur Verwendung in Aschenbechern,
   **dadurch gekennzeichnet,**
   daß sie durch Durchführung des Verfahrens nach einem der Ansprüche 1 bis 5 hergestellt ist.